# EUROPÄISCHE PATENTSCHRIFT

(11) **EP 1 903 106 B1**
(45) Veröffentlichungstag und Bekanntmachung des Hinweises auf die Patenterteilung: **14.08.2013**
(21) Anmeldenummer: 07017837.1
(22) Anmeldetag: 12.09.2007
(51) Int. Cl.: C12N 5/07, G01N 33/86, G01N 33/554

(54) **Verfahren zur Herstellung von agglutinationsfähigen Thrombozytenfragmenten und deren Verwendung**
Method for manufacturing agglutinable thrombocyte fragments and their application
Procédé de fabrication de fragments de thrombocytes pouvant s'agglutiner et leur utilisation

(30) Priorität: 25.09.2006 DE 102006045550
(43) Veröffentlichungstag der Anmeldung: 26.03.2008
(73) Patentinhaber: Siemens Healthcare Diagnostics Products GmbH, 35041 Marburg (DE)
(72) Erfinder: Patzke, Jürgen, Dr., 35043 Marburg (DE)

(56) Entgegenhaltungen:
- EP-A- 1 134 584
- US-A- 5 185 160
- KITAGUCHI T ET AL: "CHARACTERIZATION OF LIPOSOMES CARRYING VON WILLEBRAND FACTOR-BINDING DOMAIN OF PLATELET GLYCOPROTEIN IBALPHA: A POTENTIALSUBSTITUTE FOR PLATELET TRANSFUSION" BIOCHEMICAL AND BIOPHYSICAL RESEARCH COMMUNICATIONS, ACADEMIC PRESS INC. ORLANDO, FL, US, Bd. 261, Nr. 3, 11. August 1999 (1999-08-11), Seiten 784-789, XP001019106 ISSN: 0006-291X
- FEDERICI A B ET AL: "A sensitive ristocetin co-factor activity assay with recombinant glycoprotein Ib[alpha] for the diagnosis of patients with low von Willebrand factor levels" HAEMATOLOGICA, FONDAZIONE FERRATA STORTI, ROME, IT, Bd. 89, Nr. 1, 2004, Seiten 77-85, XP002390136 ISSN: 0390-6078

## Beschreibung

Die Erfindung liegt auf dem Gebiet der Diagnostik, insbesondere der Gerinnungsdiagnostik und betrifft ein Verfahren zur Herstellung von agglutinationsfähigen Thrombozytenfragmenten und deren Verwendung in diagnostischen Testverfahren, die eine Agglutinationsreaktion beinhalten, wie z. B. in einem Verfahren zur Bestimmung der VWF-Aktivität.

Der von Willebrand-Faktor (VWF) ist ein hochmolekulares, multimeres Glykoprotein, das wichtige Funktionen in dem Prozess der primären Hämostase hat. Der VWF erfüllt im Falle einer Gefäßverletzung drei wesentliche Aufgaben: Er vermittelt die Adhäsion der Thrombozyten an das Subendothel, er vermittelt die Aggregation der Thrombozyten untereinander und begünstigt dadurch die Thrombusbildung, und er bildet mit dem plasmatischen Blutgerinnungsfaktor VIII (F VIII) einen Komplex, der den FVIII vor vorzeitigem Abbau schützt. Durch Thrombin wird FVIII vom F VIII-VWF-Komplex abgespalten und zu F VIIIa aktiviert und erlangt so seine prokoagulatorische Aktivität, die auch häufig als F VIII:C bezeichnet wird.

Ein von Willebrand-Syndrom entsteht durch qualitative oder quantitative Störungen des VWF und ist eines der häufigsten erblichen Blutungsleiden. Zur Diagnose eines von Willebrand-Syndroms stehen verschiedene Screeningverfahren zur Verfügung, wie z. B. die Bestimmung der Blutungszeit (BT), quantitative Verfahren zur Bestimmung der VWF-Antigenkonzentration (VWF:Ag), wie z. B. ELISA-Verfahren sowie Verfahren zur Bestimmung der VWF-Aktivität, wie z. B. die Ristocetin-induzierte Plättchenagglutination (VWF:RCo).

Das Verfahren der Ristocetin-induzierten Plättchenagglutination, das auch als Ristocetin-Kofaktor-Test bezeichnet wird, erkennt funktionelle Defekte des VWF-Proteins, die mit quantitativen Verfahren zur Bestimmung der VWF-Antigenkonzentration nicht erkannt werden. Die Durchführung eines Ristocetin-Kofaktor-Tests zur Bestimmung der Ristocetin-Kofaktor-Aktivität ist daher für die komplette Diagnostik eines von Willebrand-Syndroms notwendig. Üblicherweise wird der Ristocetin-Kofaktor-Test durchgeführt, indem eine Plasmaprobe eines Patienten mit Thrombozyten vermischt wird. Üblicherweise werden dazu fixierte humane Thrombozyten verwendet; die Verwendung physiologisch aktiver, d. h. nativer Thrombozyten ist jedoch ebenfalls möglich. Nach Zugabe von Ristocetin aggregieren die nativen Thrombozyten bzw. agglutinieren die fixierten Thrombozyten in Abhängigkeit von dem in der Probe vorhandenen aktiven VWF. Die Aggregations- bzw. Agglutinationsreaktion kann z. B. durch Messung der Transmissionszunahme optisch erfasst werden und ermöglicht so eine Quantifizierung der VWF:RCo-Aktivität. Die Bestimmung der Aggregations- bzw. Agglutinationsreaktion ganzer Thrombozyten ist jedoch mit einigen Nachteilen verbunden.

Nachteilig ist zum einen, dass es notwendig ist, den Reaktionsansatz während der optischen Messwertaufnahme permanent zu durchmischen. Wahrscheinlich ist die dauerhafte Durchmischung nötig, um die Kollisionshäufigkeit der Thrombozyten und damit die Reaktionsgeschwindigkeit zu erhöhen. Nur wenige der im modernen Labor zur Hämostasediagnostik eingesetzten automatisierten Geräte verfügen über technische Einrichtungen, die eine solche kontinuierliche Durchmischung des Reaktionsansatzes während der Messung erlauben. Zum anderen weist der klassische Ristocetin-Kofaktor-Test eine relativ hohe Unpräzision auf. Da die große Mehrzahl der automatisierten Geräte im Bereich der Hämostasediagnostik mit optischen Einrichtungen ausgestattet ist, die nur die Messung von Wellenlängen im sichtbaren Bereich erlauben, sollte ein automatisiertes Testverfahren ebenfalls in diesem Wellenlängenbereich auswertbar sein. Bei der Messung der Transmission infolge der Thrombozytenagglutination bewirkt nur die Bildung großer Aggregate eine Transmissionszunahme im sichtbaren Wellenlängenbereich. Die Bildung großer Aggregate, die im übrigen auch anfällig sind für mechanische Zerstörung, kann jedoch in den üblicherweise relativ kleinen Testküvetten mit entsprechend kleinen optischen Messfenstern nicht präzise erfasst werden. Daher weisen die Reaktionskurven der Thrombozytenaggregation sowie der Thrombozytenagglutination typischerweise eine erhebliche Streuung der Messwerte auf.

In der Vergangenheit wurden einige alternative Testverfahren entwickelt, die eine präzisere Bestimmung der VWF:RCo-Aktivität erlauben sollen. Bei einigen diesen Verfahren kommt der VWF-Rezeptor GP1b oder Fragmente davon zum Einsatz. So wurden z. B. ELISA-Verfahren entwickelt, die die Ristocetin-induzierte Bindung des VWF an den GP1b-Rezeptor bestimmen. Ein solches Verfahren ist z. B. in Federici, A. B. et al. (Haematologica 2004; 89(1), 77-85) sowie in WO 01/02853 A2 beschrieben. Ein anderes Testformat setzt partikuläre Trägerphasen ein, wie z. B. Latexpartikel oder Liposome (z. B. Kitaguchi, T. et al., Biochem Biophys Res Commun. 1999; 261(3):784-789), an die GP1b-Protein gekoppelt ist. Die GP1b-assoziierten Partikel sollen in Gegenwart von Ristocetin und aktivem VWF agglutinieren.

Nachteilig bei den vorgenannten Verfahren ist, die zeit- und kostenaufwändige Herstellung der GP1 b-assoziierten Festphasen, der üblicherweise die Aufreinigung von nativem oder rekombinant exprimiertem GP1 b-Protein vorausgeht. Weiterhin müssen - zumindest für die Durchführung einiger Testformate - zusätzlich Antikörper, z. B. GP1b-Antikörper zur Verfügung stehen. Alles in allem handelt es sich um komplizierte Verfahren, für die eine Reihe von speziellen Reagenzien hergestellt werden müssen.

Der vorliegenden Erfindung lag die Aufgabe zugrunde, ein Mittel zur Verfügung zu stellen, das anstelle von nativen oder fixierten Thrombozyten in einem Agglutinationstest eingesetzt werden kann, und das eine präzise photometrische Bestimmung der Agglutinationsreaktion bei Wellenlängen im sichtbaren Bereich erlaubt.

Es wurde gefunden, dass Thrombozytenfragmente, die durch ein Verfahren gewonnen werden, bei dem native Thrombozyten zunächst mit Ultraschall behandelt und anschliessend fixiert werden, über eine Agglutinationsfähigkeit verfügen, die eine Verwendung der Thrombozytenfragmente in einem Agglutinationstest erlaubt.

Im Stand der Technik sind verschiedene Verfahren zur Herstellung von Thrombozytenfragmenten bekannt. Thrombozytenfragmente werden in der Literatur auch als Mikrovesikel oder Mikropartikel bezeichnet. Bei der mechanischen Herstellung von Plättchenmembranen z. B. durch Ultraschallbehandlung, Einfrieren/Auftauen oder Stickstoffkavitation entstehen Partikel unterschiedlicher Größe (Authi, K. S: Preparation of highly purified human platelet plasma and intracellular membranes using high voltage free flow electrophoresis and methods to study Ca2+ regulation. Kapitel 5 in Platelets - A Practical Approach, 1996, Hrsg. Steve P. Watson & Kalwant S. Authi, Oxford University Press, UK und Lee, D.H. & Blajchman, M.A.: Platelet Substitutes and Novel Methods of Platelet Preservation. Kapitel 60 in Platelets, 2002, Hrsg. Alan D. Michelson, Academic Press, Elsevier Science, USA). Durch die heterogene Zusammensetzung derartiger Präparationen ist die Verwendung solcher Thrombozytenfragmente in einem Agglutinationstest wenig aussichtsreich, da Partikel gleicher Größe und mit gleichen Oberflächeneigenschaften benötigt werden. Ausserdem sind die bekannten Methoden häufig zeit- und kostenaufwändig, da sie viele Arbeitsschritte umfassen. Vielfach werden die Thrombozyten mit Inhibitoren inkubiert, um eine übermäßige Aktivierung der Thrombozyten zu vermeiden und sie ausserdem vor dem Angriff abbauender Enzyme zu schützen.

Eine Methode zur Herstellung von Thrombozytenfragmenten für pharmazeutische Zwecke wird in der Patentschrift US 5,185,160 beschrieben. Dabei werden die Plättchen zunächst durch wiederholtes Einfrieren und Auftauen aufgeschlossen und schliesslich für 20 Stunden bei 60 °C zur Inaktivierung möglicher viraler Kontaminationen inkubiert. Zur Auflösung des während der Inkubation entstandenen Präzipitats wird das Präzipitat mit Ultraschall behandelt. Nach Zentrifugation der so behandelten Präparation enthält der Überstand die pharmazeutisch verträglichen Thrombozytenfragmente, die aufgrund ihrer prokoagulatorischen Eigenschaften z. B. zur Behandlung von Patienten mit einem Blutungsleiden verwendet werden können.

Gegenstand der vorliegenden Erfindung ist ein Verfahren zur Herstellung von agglutinationsfähigen Thrombozytenfragmenten, wobei native Thrombozyten zunächst mit Ultraschall behandelt werden und nach der Ultraschallbehandlung fixiert werden.

Unter dem Begriff "agglutinationsfähige Thrombozytenfragmente" sind Fragmente von Thrombozyten zu verstehen, die in Gegenwart von aktivem VWF zu einer Agglutinationsreaktion fähig sind. Die Agglutinationsfähigkeit humaner Thrombozytenfragmente kann z. B. überprüft werden, indem die Thrombozytenfragmente mit Rinderplasma zu einem Reaktionsansatz vermischt werden, wobei das Rinderplasma als Quelle für bovinen VWF dient. Eine Transmissionszunahme des Reaktionsansatzes, wobei die Transmissionszunahme bevorzugterweise mit Wellenlängen aus dem sichtbaren Bereich bestimmt wird, ist ein eindeutiges Indiz für eine stattfindende Agglutinationsreaktion und damit für die Agglutinationsfähigkeit der Thrombozytenfragmente. Alternativ können zur Überprüfung der Aggregationsfähigkeit die Thrombozytenfragmente mit Humanplasma und Ristocetin zu einem Reaktionsansatz vermischt werden.

Die Thrombozyten, die als Ausgangsmaterial in dem erfindungsgemäßen Verfahren verwendet werden, können tierischen oder menschlichen Ursprungs sein. Die Gewinnung von nativen Thrombozyten kann z. B. durch Sedimentation aus Vollblut oder plättchenreichem Plasma erfolgen. Bevorzugterweise werden die Thrombozyten vor der Ultraschallbehandlung in einer Pufferlösung resuspendiert, z. B. in einer Phosphat-, TRIS- oder Imidazol-gepufferten Lösung. Bevorzugterweise wird eine Thrombozytensuspension als Ausgangsmaterial verwendet, die etwa 0,5 bis 2 × 10⁶, besonders bevorzugt etwa 1 × 10⁶ Thrombozyten pro Mikroliter enthält.

Vorteilhafterweise enthält der Puffer, in dem die Thrombozyten resuspendiert werden, ein Antikoagulanz. Bevorzugte Antikoagulanzien sind Natriumcitrat, Citrat-Citronensäure Glucose, Saure Citronensäure Glucose, EDTA, EGTA, Heparin, Heparinderivate, synthetische Pentasaccharide, wie z. B. Fondaparinux, oder Hirudin. Das Antikoagulanz muss nicht Bestandteil des Puffers sein. Es kann der Thrombozytensuspension auch separat zugesetzt werden.

Die Ultraschallbehandlung zur Fragmentierung der nativen Thrombozyten erfolgt bevorzugt, indem eine Suspension nativer Thrombozyten ausreichend starkem Ultraschall aussetzt wird. Dies kann z. B. durch eine hohe Ultraschallintensität bei Benutzung eines Ultraschallstabes und einer entsprechend langen Dauer der Behandlung geschehen. Die notwendige Intensität und Dauer müssen mit dem jeweiligen Ultraschallgeber und den eingesetzten Volumina ermittelt werden. Bevorzugt sind Ultraschallbehandlungen, die einer Ultraschallbehandlung vergleichbar sind, die mit einem Ultraschallstab Branson Sonifier 250 (Branson Danbury, USA) und den Einstellungen Duty Cycle: 100 %, Output Control: 7 durchgeführt wird. Bevorzugterweise werden die Thrombozyten bzw. Thrombozytenfragmente vor, während und nach der Ultraschallbehandlung permanent gekühlt, z. B. durch Lagerung in einem Eisbad.

Nach der Ultraschallbehandlung werden die Thrombozytenfragmente fixiert. Vorteilhafterweise wird der Fixierungsschritt direkt im Anschluss an die Ultraschallbehandlung, d. h. ohne zeitliche Verzögerung durchgeführt. Die Fixierung der Ultraschall-behandelten Thrombozytenfragmente erfolgt bevorzugt, indem der Ultraschall-behandelten Thrombozytenfragment-Suspension ein quervernetzendes Fixierungsmittel zugegeben wird. Besonders geeignete Fixierungsmittel sind z. B. Formaldehyd, Paraformaldehyd oder Glutaraldehyd. In einer bevorzugten Ausführungsform des Verfahrens wird der Ultraschall-behandelten Thrombozytenfragment-Suspension Formaldehyd in einer Endkonzentration von 1 % zugegeben, und die Suspension wird für etwa 6 bis 16 Stunden bei etwa 2 bis 8 °C inkubiert.

In einer bevorzugten Ausführungsform werden sowohl die Ultraschallbehandlung der Thrombozyten als auch der anschliessende Fixierungsschritt in Anwesenheit eines Antikoagulanzes durchgeführt. Dies kann zum Beispiel dadurch erreicht werden, dass der Thrombozytensuspension vor der Ultraschallbehandlung wie oben beschrieben ein Antikoagulanz zugegeben wird und dann nach der Ultraschallbehandlung ohne einen Waschschritt ein Fixierungsmittel zugegeben wird.

In einer anderen bevorzugten Ausführungsform werden sowohl die Ultraschallbehandlung der Thrombozyten als auch der anschliessende Fixierungsschritt in Abwesenheit eines Thrombozytenfunktionsinhibitors durchgeführt. Thrombozytenfunktionsinhibitoren, die bevorzugterweise nicht der Thrombozytensuspension bzw. der Thrombozytenfragment-Suspension zugesetzt sind, umfassen beispielsweise Cyclooxygenase-Hemmer (wie z. B. Acetylsalicylsäure), Phosphodiesterase-Inhibitoren (wie z. B. Theophyllin, Koffein, Dipyridamol, Milrinon, Cilostamid, Zaprinast), den cAMP-Spiegel erhöhende Agenzien (wie z. B. Prostaglandin E1, Prostaglandin 12, Prostacyclin, Adenosin) sowie ADP-abbauende Enzyme (wie z. B. Apyrase).

Nach der Fixierung wird das Fixierungsmittel typischerweise aus der Suspension entfernt, z. B. durch eine Dialyse, bevorzugterweise mit einem Phosphatpuffer. Bevorzugterweise wird eine Dialyse solange durchgeführt, bis die Konzentration des Fixierungsmittels weniger als 0,001 % in der Suspension beträgt.

In einer bevorzugten Ausführungsform werden besonders vorteilhafte Fraktionen der erfindungsgemäß hergestellten Thrombozytenfragmente durch Zentrifugation der Thrombozytenfragment-Suspension angereichert. Die Anreicherung kann dadurch erfolgen, dass die Thrombozytenfragment-Suspension in einem ersten Zentrifugationsschritt bei 1500 g für 10 Minuten zentrifugiert wird, und der resultierende Überstand in einem zweiten Zentrifugationsschritt bei 4000 g für 40 Minuten zentrifugiert wird. Der Überstand wird verworfen und die sedimentierten Thrombozytenfragmente werden in einer Pufferlösung, bevorzugt einem Phosphatpuffer resuspendiert. Bevorzugterweise werden die Zentrifugationsschritte bei einer Temperatur von etwa 20 °C durchgeführt.

Eine weitere Ausführungsform des erfindungsgemäßen Verfahrens zur Herstellung von agglutinationsfähigen Thrombozytenfragmenten umfasst einen weiteren Verfahrensschritt, bei dem nach dem Fixierungsschritt GP1 b-Rezeptor-Protein an die Thrombozytenfragmente gebunden wird. GP1b ist ein Glykoprotein, das ein integraler Bestandteil der Thrombozytenmembran ist und an der Bindung des VWF an die Thrombozytenoberfläche beteiligt ist. Bei dem GP1b-Protein, das an die fixierten Thrombozytenfragmente gebunden wird, kann es sich um natives, d. h. aus natürlichen Quellen, wie z. B. aus Plasma, isoliertes GP1b-Protein oder um rekombinant hergestelltes GP1b-Protein handeln. Im Sinne der vorliegenden Erfindung umfasst der Begriff "GP1b-Protein" sowohl das vollständige native oder rekombinante Protein als auch Fragmente davon, insbesondere N-terminale Fragmente, die die Aminosäurereste 1-290 (GP1bα-Untereinheit) umfassen.

Zur Bindung des GP1b-Proteins an die Thrombozytenfragmente können z. B. ein oder mehrere Antikörper verwendet werden, an die GP1b-Protein gekoppelt wurde und die an Thrombozyten-Oberflächenepitope, wie Proteine oder Lipide binden. Geeignete Thrombozyten-Oberflächenepitope zur Bindung von an GP1b gekoppelten Antikörpern sind beispielsweise GPIIb, GPllla, GPla, GPVI, Phosphatidylcholin und/oder Phosphatidylserin. Bevorzugterweise werden GP1b-gekoppelte Antikörper verwendet, deren GP1b-Anteil weiterhin in der Lage ist, VWF zu binden. Zur Kopplung von GP1b an geeignete Antikörper kann eine Vielzahl bekannter Methoden verwendet werden. Zur stabilen Bindung eines Antikörper-GP1 b-Komplexes an die Oberfläche der Thrombozytenfragmente können ebenfalls diverse bekannte Methoden verwendet, die eine Quervernetzung mit Proteinen oder anderen Molekülen der Thrombozytenfragmentoberfläche ermöglichen.

Das GP1b-Protein kann auch chemisch an die Thrombozytenfragmente gebunden werden oder durch die Verwendung von Vermittlermolekülen, wie z. B. Proteinen, die eine Verbindung zwischen dem GP1b-Protein und einem Molekül der Fragmentoberfläche herstellen. Beispielsweise kann das GP1 b-Protein an Annexin V gekoppelt werden, welches an Phosphatidylserin bindet. Phosphatidylserin ist besonders bei aktivierten Thrombozyten und den daraus entstandenen Fragmenten auf der Oberfläche zu finden. Weitere Beispiele für Vermittlermoleküle sind die dem Fachmann bekannten "Crosslinker"-Moleküle, wie z. B. Bis-Sulfosuccinimidylsuberat (Polyethylenglykol 5), Bis-N-succinimidyl-pentaethylenglykolester und viele andere mehr, mit denen Proteine miteinander verknüpft werden können. Die Verwendung von Vermittlermolekülen kann den vorteilhaften Effekt haben, den GP1b-Rezeptor weit von der Oberfläche entfernt zu halten, wodurch er leichter den VWF binden kann.

Die vorliegende Erfindung betrifft weiterhin die Verwendung der erfindungsgemäß hergestellten agglutinationsfähigen Thrombozytenfragmente in einem Verfahren zur Bestimmung der VWF-Aktivität einer Probe.

Ein weiterer Gegenstand der vorliegenden Erfindung ist ein Verfahren zur Bestimmung der VWF-Aktivität einer Probe, wobei die Probe mit den erfindungsgemäß hergestellten, agglutinationsfähigen Thrombozytenfragmenten versetzt wird und die Agglutinationsreaktion der Thrombozytenfragmente im Reaktionsansatz bestimmt wird. Dazu wird die Probe, bevorzugterweise eine Plasmaprobe, mit den Thrombozytenfragmenten, die bevorzugterweise in einem Puffer suspendiert sind, zu einem Reaktionsansatz vermischt. Zur Bestimmung der Ristocetin-abhängigen VWF-Aktivität kann dem Reaktionsansatz zusätzlich Ristocetin zugegeben werden.

In einer besonders bevorzugten Ausführungsform eines erfindungsgemäßen Verfahrens zur Bestimmung der VWF-Aktivität einer Probe wird den Thrombozytenfragmenten, kurz bevor sie mit der Probe vermischt werden, Natriumchlorid, bevorzugt in Form einer NaCl-Lösung zugegeben. Bevorzugterweise beträgt die NaCl-Konzentration in der Thrombozytenfragment-Suspension nach Zugabe von NaCl unter 5 g/L, besonders bevorzugt unter 3 g/L.

In einer weiteren vorteilhaften Ausführungsform eines erfindungsgemäßen Verfahrens zur Bestimmung der VWF-Aktivität wird der Reaktionsansatz nicht kontinuierlich durchmischt. Überraschenderweise ist eine kontinuierliche Durchmischung des Reaktionsansatzes während der Messwertaufnahme, wie z. B. durch Rühren oder Schütteln des Testansatzes nicht erforderlich, da auch ohne kontinuierliche Durchmischung des Reaktionsansatzes eine Agglutinationsreaktion erfolgt, die eine Quantifizierung der VWF-Aktivität ermöglicht.

Bevorzugterweise wird die Agglutinationsreaktion der Thrombozytenfragmente im Reaktionsansatz durch Messung einer optischen Eigenschaft des Reaktionsansatzes, die sich in Abhängigkeit von der Agglutinationsreaktion verändert, gemessen. Besonders bevorzugt wird die Transmissionsabnahme oder die Zunahme der Lichtstreuung gemessen.

### Abbildungen

### Fig. 1

Der lineare Anstieg der Referenzkurve zeigt: die Agglutinationsreaktion der erfindungsgemäßen Thrombozytenfragmente verhält sich proportional zur VWF-Aktivität in Rinderplasma.

### Fig. 2

Korrelation der Testergebnisse (VWF-Aktivität), die mit einem konventionellen Thrombozyten-Test und mit dem neuen Thrombozytenfragmente-Test für dieselben Rinderplasmaproben bestimmt wurden. Der Korrelationskoeffizient von R² = 0,9879 bestätigt den linearen Zusammenhang zwischen den Testergebnissen, der beiden Teste.

### Ausführungsbeispiele

### Beispiel 1: Herstellung von agglutinationsfähigen Thrombozytenfragmenten

Zur Gewinnung nativer Thrombozyten wurde ein Thrombozyten-Apherese-Konzentrat aus humanem Vollblut in mehreren Probengefäßen in einer vorgekühlten Zentrifuge für 10 Minuten bei 1500 g ohne Bremse bei 4°C zentrifugiert. Die Überstände wurden abgenommen und die Sedimente in jeweils 30 mL gekühltem Citrat-Puffer (9,07 g/L Di-Natriumhydrogenphosphat, 1,65 g/L Kaliumdihydrogenphosphat, 0,38 % Natrium-Citrat, 1,2 g/L NaCl) aufgenommen. Die Resuspension und Homogenisierung erfolgte mit Hilfe eines Ultra Turrax-Dispergiergerätes (Janke und Kunkel GmbH, Staufen, Deutschland) (10 sec bei 13500 U/min). Anschließend wurde die Thrombozytenzahl durch Verdünnung mit Citrat-Puffer auf 1.000.000 Thrombozyten/µL eingestellt.

Die Thrombozytensuspension wurde für 30 Minuten im Eisbad inkubiert. Danach wurden 20 mL-Aliquots mit dem Ultraschallstab Branson Sonifier 250 (Branson, Danbury, USA) bei permanenter Kühlung auf Eis 15 Sekunden lang folgendermaßen behandelt: Duty Cycle: 100 %, Output Control: 7. Direkt im Anschluss wurde Formaldehyd zugegeben, so dass die Endkonzentration von Formaldehyd 1 % betrug. Nach einer Fixierung über Nacht (für etwa 14 Stunden) bei 2-8 °C erfolgte eine Dialyse mit Puffer (9,07 g/L Di-Natriumhydrogenphosphat, 1,65 g/L Kaliumdihydrogenphosphat, 1,2 g/L NaCl), bis der Formaldehyd-Gehalt auf eine Konzentration von weniger als 0,001 % gesunken war.

Zur Anreicherung besonders vorteilhafter Fraktionen wurde die Fragmentsuspension mit Hilfe des Ultra Turrax-Dispergiergerätes homogenisiert und in 20 mL-Aliquots für 10 Minuten bei 1500 g bei 20 °C zentrifugiert. Die Überstände wurden abgenommen und für 40 Minuten bei 4000 g bei 20 °C zentrifugiert. Die Sedimente wurden in jeweils 1 mL Phosphatpuffer (0,907 g/L Di-Natriumhydrogenphosphat, 0,165 g/L Kaliumdihydrogenphosphat) aufgenommen und mit dem Schüttler solange bearbeitet, bis sich das Sediment vollständig vom Boden abgelöste. Mehrere auf diese Art resuspendierte Sedimente wurden in einem 14 mL-Kunststoffröhrchen vereinigt und mit Hilfe des Ultra Turrax-Dispergiergerätes für 5 bis 10 Sekunden bei 13500 Umdrehungen/Minute homogenisiert.

Direkt vor dem Einsatz der Fragmentsuspension zur Bestimmung der VWF-Aktivität erfolgte die Zugabe von NaCl, so dass die NaCl-Konzentration in der Suspension 2 g/L betrug.

### Beispiel 2: Bestimmung der VWF-Aktivität in Rinderplasma unter Verwendung der erfindungsgemäß hergestellten Thrombozytenfragmente

Folgende Schritte wurden automatisch auf dem Gerinnungsmessgerät BCT^{®} (Dade Behring Marburg GmbH, Marburg, Deutschland) durchgeführt:

50 µL plättchenarmes Rindercitratplasma wurde mit 100 µL einer wie in Beispiel 1 hergestellten Thrombozytenfragment-Suspension zu einem Reaktionsansatz vermischt und für 15 Sekunden bei 37 °C inkubiert.

Die Extinktion des Reaktionsansatzes bei 620 nm wurde über einen Zeitraum von 90 Sekunden gemessen, ohne dass der Reaktionsansatz während der Messung durchmischt wurde. Anhand der so ermittelten Kinetik der Aggregationsreaktion wurde die Extinktionsänderung (mE/Minute) bestimmt, und es wurde die maximale Extinktionsänderung (Vmax der Extinktionszunahme) ermittelt.

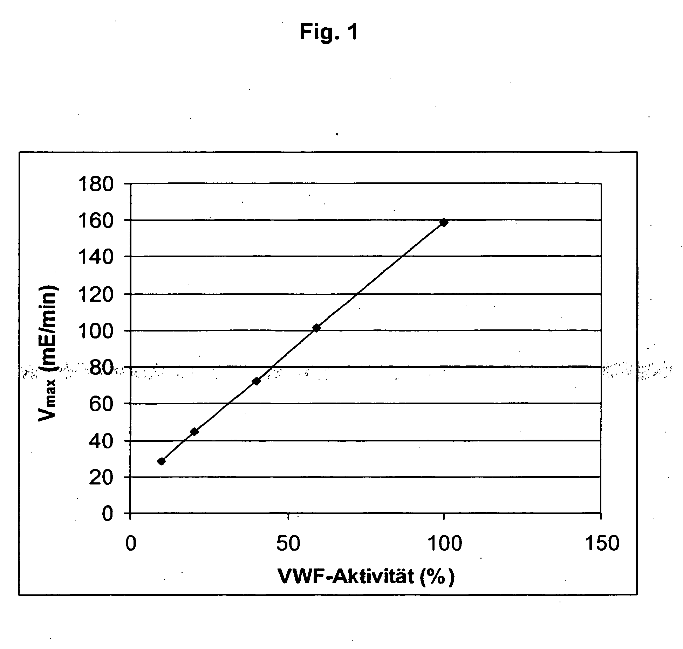

Zur Erstellung einer Kalibrationskurve wurde normales Rinderplasma (VWF-Aktivität = 100 %) mit VWF-Mangelplasma in einer Verdünnungsreihe verdünnt. Das unverdünnte normale Plasma sowie die verschiedenen Verdünnungen, die unterschiedliche VWF-Aktivitäten aufwiesen, wurden mit dem beschriebenen Verfahren analysiert. Fig. 1 zeigt die auf diese Weise erstellte Referenzkurve. Die Agglutinationsreaktion der erfindungsgemäßen Thrombozytenfragmente verhält sich proportional zur VWF-Aktivität einer Probe.

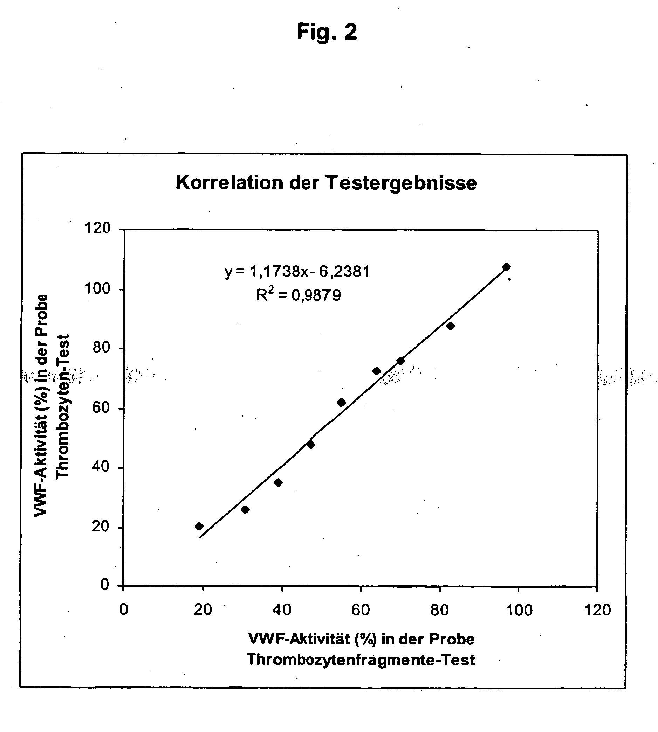

In einem Vergleichsexperiment wurden dieselben Rinderplasmaverdünnungen in einem konventionellen Agglutinationstest getestet, bei dem fixierte ganze Thrombozyten mit der Probe vermischt wurden. Wie aus Fig. 2 hervorgeht, korrelieren die Ergebnisse des neuen Testverfahrens, bei dem die erfindungsgemäß hergestellten Thrombozytenfragmente verwendet werden, sehr gut mit den Testergebnissen des konventionellen Tests.

## Patentansprüche

1. Verfahren zur Herstellung von agglutinationsfähigen Thrombozytenfragmenten **dadurch gekennzeichnet, dass** native Thrombozyten zunächst mit Ultraschall behandelt werden und anschließend fixiert werden.

2. Verfahren gemäß Anspruch 1 **dadurch gekennzeichnet, dass** die Thrombozyten in Anwesenheit eines Antikoagulanzes mit Ultraschall behandelt und fixiert werden.

3. Verfahren gemäß einem der Ansprüche 1 bis 2 **dadurch gekennzeichnet, dass** die Thrombozyten in Abwesenheit von Thrombozytenfunktions-Inhibitoren mit Ultraschall behandelt und fixiert werden.

4. Verfahren gemäß einem der Ansprüche 1 bis 3 **dadurch gekennzeichnet, dass** die Thrombozyten nach der Ultraschallbehandlung mit einem Fixierungsmittel aus der Gruppe Formaldehyd, Paraformaldehyd und Glutaraldehyd fixiert werden.

5. Verfahren gemäß einem der Ansprüche 1 bis 4 **dadurch gekennzeichnet, dass** nach der Fixierung GP1b-Rezeptor-Protein an die Thrombozytenfragmente gebunden wird.

6. Verwendung agglutinationsfähiger Thrombozytenfragmente, die nach einem der Ansprüche 1-5 erhältlich sind, in einem Verfahren zur Bestimmung der VWF-Aktivität einer Probe.

7. Verfahren zur Bestimmung der VWF-Aktivität einer Probe **dadurch gekennzeichnet, dass** die Probe mit agglutinationsfähigen Thrombozytenfragmenten, die nach einem der Ansprüche 1-5 erhältlich sind, versetzt wird und die Agglutinationsreaktion der Thrombozytenfragmente im Reaktionsansatz bestimmt wird.

8. Verfahren gemäß Anspruch 7 **dadurch gekennzeichnet, dass** zu dem Reaktionsansatz Ristocetin gegeben wird.

9. Verfahren gemäß einem der Ansprüche 7 und 8 **dadurch gekennzeichnet, dass** die Thrombozytenfragmente in Form einer Suspension eingesetzt werden, die Natriumchlorid in einer Konzentration von weniger als 5 g/L, bevorzugt unter 3 g/L enthält.

10. Verfahren gemäß einem der Ansprüche 7 bis 9 **dadurch gekennzeichnet, dass** der Reaktionsansatz nicht kontinuierlich durchmischt wird.

11. Verfahren gemäß einem der Ansprüche 7 bis 10 **dadurch gekennzeichnet, dass** die Agglutinationsreaktion der Thrombozytenfragmente im Reaktionsansatz durch Messung der Transmissionsabnahme bestimmt wird.

## Claims

1. A method for preparing agglutinatable platelet fragments which comprises native platelets being first treated with ultrasound and then fixed.

2. The method as claimed in claim 1, wherein the platelets are treated with ultrasound and fixed in the presence of an anticoagulant.

3. The method as claimed in either of claims 1 to 2, wherein the platelets are treated with ultrasound and fixed in the absence of platelet function inhibitors.

4. The method as claimed in any of claims 1 to 3, wherein the platelets are fixed after the ultrasound treatment with a fixative from the group of formaldehyde, paraformaldehyde and glutaraldehyde.

5. The method as claimed in any of claims 1 to 4, wherein, after the fixation, GP1b receptor protein is bound to the platelet fragments.

6. The use of agglutinatable platelet fragments which can be obtained in accordance with any of claims 1 to 5 in a method for determining the VWF activity in a sample.

7. A method for determining the VWF activity in a sample, which comprises mixing the sample with agglutinatable platelet fragments which can be obtained in accordance with any of claims 1 to 5, and determining the agglutination reaction of the platelet fragments in the reaction mixture.

8. The method as claimed in claim 7, wherein ristocetin is added to the reaction mixture.

9. The method as claimed in either of claims 7 and 8, wherein the platelet fragments are employed in the form of a suspension which comprises sodium chloride in a concentration of less than 5 g/l, preferably below 3 g/l.

10. The method as claimed in any of claims 7 to 9, wherein the reaction mixture is not continuously mixed.

11. The method as claimed in any of claims 7 to 10, wherein the agglutination reaction of the platelet fragments in the reaction mixture is determined by measuring the decrease in transmittance.

## Revendications

1. Procédé de réparation de fragments de thrombocyte aptes à s'agglutiner, **caractérisé en ce que** l'on traite des thrombocytes naturels d'abord aux ultrasons et ensuite on les immobilise.

2. Procédé suivant la revendication 1,
**caractérisé en ce que** l'on traite les thrombocytes aux ultrasons en la présence d'un anticoagulant et on les immobilise.

3. Procédé suivant l'une des revendications 1 ou 2,
**caractérisé en ce que** l'on traite les thrombocytes aux ultrasons en l'absence d'inhibiteur de fonction de thrombocyte et on les immobilise.

4. Procédé suivant l'une des revendications 1 à 3,
**caractérisé en ce que** l'on immobilise les thrombocytes, après le traitement aux ultrasons, par un agent d'immobilisation choisi dans le groupe du formaldéhyde, du paraformaldéhyde et du glutaraldéhyde.

5. Procédé suivant l'une des revendications 1 à 4,
**caractérisé en ce que** l'on fixe, après l'immobilisation, la protéine de récepteur GP1B aux fragments de thrombocyte.

6. Utilisation de fragments de thrombocyte aptes à s'agglutiner, qui peuvent être obtenus suivant l'une des revendications 1 à 5 dans un procédé de détermination de l'activité VWF d'un échantillon.

7. Procédé de détermination de l'activité VWF d'un échantillon, **caractérisé en ce que** on mélange l'échantillon à des fragments de thrombocyte qui sont aptes à s'agglutiner et qui peuvent être obtenus suivant l'une des revendications 1 à 5 et on détermine la réaction d'agglutination des fragments de thrombocyte dans la masse réactionnelle.

8. Procédé suivant la revendication 7,
**caractérisé en ce qu'**on ajoute de la ristocétine à la masse réactionnelle.

9. Procédé suivant l'une des revendications 7 et 8,
**caractérisé en ce que** l'on engage les fragments de thrombocyte sous la forme d'une suspension qui contient du chlorure de sodium en une concentration de moins de 5g/L, de préférence inférieure à 3g/L.

10. Procédé suivant l'une des revendications 7 à 9,
**caractérisé en ce que** l'on ne mélange pas continuellement la masse réactionnelle.

11. Procédé suivant l'une des revendications 7 à 10,
**caractérisé en ce que** l'on détermine la réaction d'agglutination des fragments de thrombocyte dans la masse réactionnelle en mesurant la diminution de la transmission.
